# EUROPÄISCHE PATENTSCHRIFT

(11) **EP 1 450 003 B1**
(45) Veröffentlichungstag und Bekanntmachung des Hinweises auf die Patenterteilung: **25.05.2011**
(21) Anmeldenummer: 04002383.0
(22) Anmeldetag: 04.02.2004
(51) Int. Cl.: E21B 37/06, E21B 41/02, C23F 11/14, C10L 3/06, C07C 227/00, C07C 229/02, C07C 233/00

(54) **Korrosions- und Gashydratinhibitoren mit verbesserter Wasserlöslichkeit und erhöhter biologischer Abbaubarkeit**
Corrosion and gas hydrate inhibitors with improved water solubility and biodegradability
Inhibiteurs de corrosion et de formation d'hydrates de gaz, à solubilité dans l'eau et biodégradabilité améliorées

(30) Priorität: 24.02.2003 DE 10307728
(43) Veröffentlichungstag der Anmeldung: 25.08.2004
(73) Patentinhaber: Clariant Produkte (Deutschland) GmbH, 65929 Frankfurt am Main (DE)
(72) Erfinder: Dahlmann, Uwe, Dr., 69126 Heidelberg (DE); Feustel, Michael, Dr., 55278 Köngernheim (DE)

(56) Entgegenhaltungen:
- EP-A- 0 359 048
- WO-A-98/23792

## Beschreibung

Die vorliegende Erfindung betrifft ein Additiv und ein Verfahren zur Korrosionsinhibierung und Gashydratinhibierung an und in Einrichtungen zur Förderung und Transport von Kohlenwasserstoffen in der Erdölförderung und -verarbeitung.

In technischen Prozessen, bei denen Metalle mit Wasser oder auch mit Öl-Wasser-Zweiphasensystemen in Kontakt kommen, besteht die Gefahr der Korrosion. Die ist besonders ausgeprägt, wenn die wässrige Phase wie bei Erdölgewinnungs- und Verarbeitungsprozessen stark salzhaltig oder durch gelöste Sauergase, wie Kohlendioxid bzw. Schwefelwasserstoff, azid ist. Daher ist die Ausbeutung einer Lagerstätte und die Verarbeitung von Erdöl ohne spezielle Additive zum Schutz der eingesetzten Ausrüstungen nicht möglich.

Geeignete Korrosionsschutzmittel für die Erdölförderung und -verarbeitung sind zwar schon seit langem bekannt, jedoch aus Gründen des Umweltschutzes für Offshore-Anwendungen zukünftig inakzeptabel.

Als typische Korrosionsinhibitoren des Standes der Technik besitzen Amide, Amidoamine bzw. Imidazoline von Fettsäuren und Polyaminen eine äußerst gute Öllöslichkeit und sind somit in der korrosiven Wasserphase aufgrund schlechter Verteilungsgleichgewichte (Partitioning) nur in geringer Konzentration vorhanden. Demgemäss müssen diese Produkte trotz ihrer schlechten biologischen Abbaubarkeit in hoher Dosierung eingesetzt werden.

DE-A-199 30 683 beschreibt entsprechende Amidoamine/lmidazoline, die durch Umsetzung von Alkylpolyglykolethercarbonsäuren mit Polyaminen erhalten werden, und die aufgrund eines besseren Partitioning in geringeren Konzentrationen eingesetzt werden können.

Quartäre Alkylammoniumverbindungen (Quats) stellen alternative Korrosionsschutzmittel des Standes der Technik dar, die neben den korrosionsinhibierenden auch biostatische Eigenschaften besitzen können. Trotz einer verbesserten Wasserlöslichkeit zeigen die Quats, zum Beispiel im Vergleich zu den Imidazolinen, eine deutlich reduzierte Filmpersistenz und führen daher ebenfalls nur in höherer Dosierung zu einem effektiven Korrosionsschutz. Die starke Algentoxizität und die mäßige biologische Abbaubarkeit beschränken den Einsatz von Quats immer mehr auf ökologisch unsensible Anwendungsgebiete, z.B. Onshore.

EP-B-0 946 788 beschreibt ein Verfahren zum Schutz von Metalloberflächen gegenüber Korrosion unter Verwendungen von Esterquats, von denen eine gute biologische Abbaubarkeit und eine geringe Aquatoxizität offenbart wird.

EP-A-0 320 769 offenbart gegebenenfalls quaternierte Fettsäureester von oxalkylierten Alkylamino-alkylenaminen und deren Verwendung als Korrosionsinhibitor.

EP-B-0 212 265 beschreibt quartäre Polykondensate aus alkoxylierten Alkylaminen und Dicarbonsäuren und deren Verwendung als Korrosionsinhibitor und Spalter in Rohölen.

EP-B-0 446 616 beschreibt Ampholyte und Betaine auf Basis Fettsäure-Amidoalkylaminen, die unter den gegebenen Testbedingungen einen sehr guten Korrosionsschutz und eine wesentlich reduzierte Algentoxizität aufweisen.

EP-B-0 584 711 offenbart Ester, Amide bzw. Imide von Alkenylbernsteinsäuren mit Alkoxyalkylaminen und deren Metall- bzw. Ammoniumsalze als Emulgatoren und Korrosionsinhibitoren für Metallbearbeitungshilfsmittel. Die Verwendung von Alkenylbernsteinsäureester- bzw. amidoaminquats oder entsprechenden Betainen ist nicht beschrieben.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25°C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck- und Temperaturverhältnissen zu Problemen führen, indem sich in den Bohrspülflüssigkeiten Gashydrate bilden.

Um solche Probleme zu vermeiden, kann die Gashydratbildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der Offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Anti-Agglomerates). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Agglomeratinhibitoren darstellen. Von besonderer Bedeutung sind hierbei Polymere mit Kohlenstoff-Backbone, die in den Seitengruppen sowohl cyclische (Pyrrolidon- oder Caprolactamreste) als auch acyclische Amidstrukturen enthalten.

EP-B-0 736 130 offenbart ein Verfahren zur Inhibierung von Gashydraten, welches die Zuführung einer Substanz der Formel 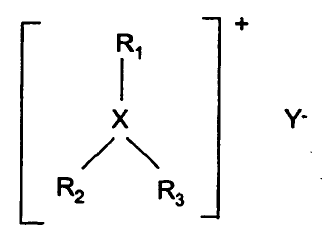 mit X = S, N - R₄ oder P - R₄, R₁, R₂ und R₃ = Alkyl mit mindestens 4 Kohlenstoffatomen, R₄ = H oder ein organischer Rest, und Y = Anion erfordert.

Umfasst werden also Verbindungen der Formel 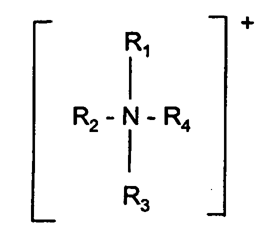 worin R₄ ein beliebiger Rest sein kann, die Reste R₁ bis R₃ aber Alkylreste mit mindestens 4 Kohlenstoffatomen darstellen müssen.

EP-B-0 824 631 offenbart ein Verfahren zur Inhibierung von Gashydraten, welches die Zuführung einer Substanz der Formel 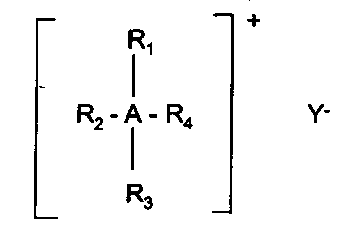 mit R₁, R₂ = gerade/verzweigte Alkylreste mit 4 oder 5 Kohlenstoffatomen, R₃, R₄ = organische Reste mit mindestens 8 Kohlenstoffatomen und A = Stickstoff oder Phosphor erfordert. Y- ist ein Anion. Es müssen zwei der Reste R₁ bis R₄ gerade oder verzweigte Alkylreste mit 4 oder 5 Kohlenstoffatomen sein.

US-5 648 575 offenbart ein Verfahren zur Inhibierung von Gashydraten. Das Verfahren umfasst die Verwendung einer Verbindung der Formel 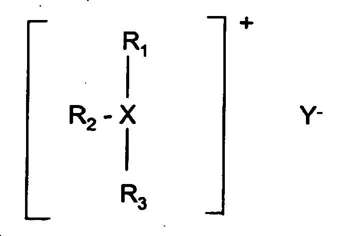 worin R¹, R² gerade oder verzweigte Alkylgruppen mit mindestens 4 Kohlenstoffatomen, R³ ein organischer Rest mit mindestens 4 Atomen, X Schwefel, NR⁴ oder PR⁴, R⁴ Wasserstoff oder ein organischer Rest, und Y ein Anion sind. Das Dokument offenbart nur solche Verbindungen, die mindestens zwei Alkylreste mit mindestens 4 Kohlenstoffatomen aufweisen.

US-6 025 302 offenbart Polyetheramin-ammoniumverbindungen als Gashydratinhibitoren, deren Ammoniumstickstoffatom neben der Polyetheraminkette 3 Alkylsubstituenten trägt.

WO-99/13197 offenbart Ammoniumverbindungen als Gashydratinhibitoren, die wenigstens eine mit Alkylcarbonsäuren veresterte Alkoxygruppe besitzen. Die Vorteile einer Verwendung von Alkenylbersteinsäurederivaten ist nicht offenbart.

WO-01/09082 offenbart ein Verfahren zur Herstellung von quartären Aminen, die jedoch keine Alkoxygruppen tragen, und deren Verwendung als Gashydratinhibitoren.

WO-00/078 706 offenbart quartäre Ammoniumverbindungen als Gashydratinhibitoren, die jedoch keine Carbonylreste tragen.

EP-B-0 914 407 offenbart die Verwendung von trisubstituierten Aminoxiden als Gashydratinhibitoren.

US-5 254 138 offenbart Detergent-Additive für Dieselkraftstoff, die Succinimid-Polyaminderivate umfassen.

Aufgabe der vorliegenden Erfindung war es, neue Korrosionsinhibitoren zu finden, die bei konstant gutem oder verbessertem Korrosionsschutz neben einer optimierten Wasserlöslichkeit, einer schnelleren Filmbildung und somit verbesserten Filmpersistenz auch eine verbesserte biologische Abbaubarkeit im Vergleich zu den Korrosionsinhibitoren des Standes der Technik bieten.

Aufgabe der vorliegenden Erfindung war es ferner, verbesserte Additive zu finden, die sowohl die Bildung von Gashydraten verlangsamen (kinetische Inhibitoren) als auch Gashydratagglomerate klein und pumpbar halten (Anti-Agglomerates), um so ein breites Anwendungsspektrum mit hohem Wirkpotential zu gewährleisten. Des weiteren sollten die derzeit verwendeten thermodynamischen Inhibitoren (Methanol und Glykole), die beträchtliche Sicherheitsprobleme und Logistjkprobleme verursachen, ersetzt werden können.

Gashydratinhibitoren des Standes der Technik werden gewöhnlich mit Korrosionsinhibitoren co-additiviert, um Korrosion der Transport- und Fördereinrichtungen vorzubeugen. Aufgrund der häufig nicht unmittelbar gegebenen Verträglichkeit von Gashydratinhibitor und Korrosionsschutzmittel bei der Formulierung ergibt sich daraus für den Anwender ein zusätzlichen Aufwand. Ein wesentlicher Vorteil gegenüber dem Stand der Technik bestände, wenn eine Co-Additivierung mit Korrosionsinhibitoren nicht mehr zwingend erforderlich ist.

Es wurde nun überraschenderweise gefunden, dass quartäre Alkylamino-alkylester bzw. quartäre Alkylamino-alkylamide, gegebenenfalls enthaltend quartäre Alkylamino-alkylimide, von Alkenylbernsteinsäuren eine ausgezeichnete Wirkung als Korrosionsinhibitoren und Gashydratinhibitoren aufweisen, sowie eine verbesserte Filmpersistenz und gute biologische Abbaubarkeit zeigen.

Gegenstand der Erfindung ist somit die Verwendung von Verbindungen der Formel (1) 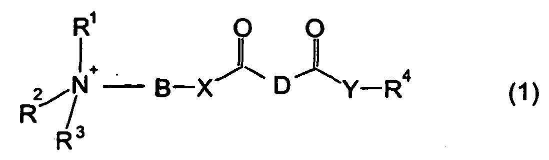 worin
- R¹, R²: unabhängig voneinander C₁- bis C₂₂-Alkyl, C₂- bis C₂₂-Alkenyl, C₆- bis C₃₀-Aryl- oder C₇- bis C₃₀-Alkylaryl, und,
- R³: C₁- bis C₂₂-Alkyl, C₂- bis C₂₂-Alkenyl, C₆- bis C₃₀-Aryl- oder C₇- bis C₃₀-Alkylaryl,-CHR⁵-COO- oder -O⁻,
- R⁴: M, Wasserstoff oder einen gegebenenfalls Heteroatome enthaltenden organischen Rest mit 1 bis 100 Kohlenstoffatomen,
- B: eine gegebenenfalls substituierte C₁- bis C₁₀-Alkylengruppe,
- D: eine mit einem organischen Rest mit 1 bis 600 Kohlenstoffatomen substituierte Ethylengruppe,
- X, Y: unabhängig voneinander O oder NR⁶,
- R⁵, R⁶: unabhängig voneinander Wasserstoff, C₁- bis C₂₂-Alkyl, C₂- bis C₂₂-Alkenyl, C₆- bis C₃₀-Aryl- oder C₇- bis C₃₀-Alkylaryl, und
- M: ein Kation
bedeuten, als Korrosions- und Gashydratinhibitoren.

Ein weiterer Gegenstand der Erfindung ist ein Verfahren zur Inhibierung von Korrosion an Metalloberflächen, insbesondere von eisenhaltigen Metallen, indem einem korrosiven System, welches mit den Metalloberflächen in Kontakt steht, mindestens eine Verbindung der Formel (1) zugesetzt wird.

Ein weiterer Gegenstand der Erfindung ist ein Verfahren zur Inhibierung von Gashydraten, indem einem zur Bildung von Gashydraten neigenden System aus Wasser und Kohlenwasserstoffen mindestens eine Verbindung der Formel (1) zugesetzt wird.

Ein weiterer Gegenstand der Erfindung sind die Verbindungen der Formel (1).

Korrosive Systeme im Sinne dieser Erfindung sind bevorzugt flüssig/flüssig- bzw. flüssig/gasförmig-Mehrphasensysteme, bestehend aus Wasser und Kohlenwasserstoffen, die in freier und/oder gelöster Form korrosive Bestandteile, wie Salze und Säuren, enthalten. Die korrosiven Bestandteile können auch gasförmig sein, wie etwa Schwefelwasserstoff und Kohlendioxid.

Kohlenwasserstoffe im Sinne dieser Erfindung sind organische Verbindungen, die Bestandteile des Erdöls/Erdgases sind, und deren Folgeprodukte. Kohlenwasserstoffe im Sinne dieser Erfindung sind auch leichtflüchtige Kohlenwasserstoffe, wie beispielsweise Methan, Ethan, Propan, Butan. Für die Zwecke dieser Erfindung zählen dazu auch die weiteren gasförmigen Bestandteile des Erdöls/Erdgases, wie etwa Schwefelwasserstoff und Kohlendioxid.

B kann geradkettig oder verzweigt sein und steht vorzugsweise für eine C₂- bis C₄-Alkylengruppe, insbesondere für eine Ethylen- oder Propylengruppe. B kann gegebenenfalls mit funktionellen Gruppen substituiert sein, vorzugsweise mit einer oder mehreren OH-Gruppen.

R¹ und R² stehen vorzugsweise unabhängig voneinander für eine Alkyl- oder Alkenylgruppe von 2 bis 14 Kohlenstoffatomen, insbesondere für solche Gruppen mit 3 bis 8 Kohlenstoffatomen und speziell für Butyl-Gruppen.

R³ steht vorzugsweise für eine Alkyl- oder Alkenylgruppe mit 1 bis 12 Kohlenstoffatomen, insbesondere mit 1 bis 4 Kohlenstoffatomen.

R⁵ und R⁶ stehen vorzugsweise für Wasserstoff.

R⁴ kann ein beliebiger organischer Rest sein, der 1 bis 100 C-Atome enthält, und der gegebenenfalls Heteroatome enthalten kann. Enthält R⁴ Heteroatome, so handelt es sich vorzugsweise um Stickstoff- oder Sauerstoffatome oder beides, vorzugsweise um beides. Die Stickstoffatome können in quaternierter Form vorliegen.

R⁴ steht vorzugsweise für einen Rest der Formel (2) 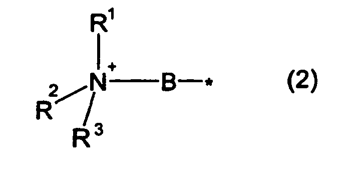 worin R¹, R², R³ und B die bereits oben angegebenen Bedeutungen mit den jeweils oben für R¹, R², R³ und B angegebenen Vorzugsbereichen haben.

In einer weiteren bevorzugten Ausführungsform umfasst R⁴ Wasserstoff, der sowohl kovalent gebunden als auch dissoziert vorliegen kann.

D bildet mit den Carbonylgruppen, an die es gebunden ist, ein substituiertes Bernsteinsäurederivat. D stellt daher eine Struktureinheit der Formel 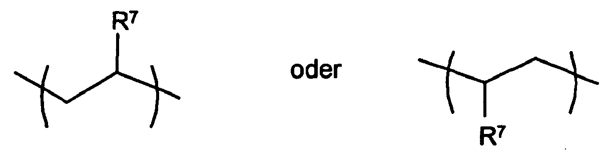 dar, in der R⁷ für einen beliebigen, gegebenenfalls Heteroatome enthaltenden organischen Rest mit 1 bis 600 Kohlenstoffatome, insbesondere für C₂- bis C₁₀₀-Alkyl- oder Alkenylreste steht. Die Alkenylreste R⁷ können von C₂- bis C₈-Alkenen durch Oligomerisierung abgeleitet sein, insbesondere aus Ethylen, Propylen oder Butylen.

Enthält R⁷ Heteroatome, so handelt es sich vorzugsweise um Stickstoff- oder Sauerstoffatome oder beides, vorzugsweise um beides. Die Stickstoffatome können in quaternierter Form vorliegen.

Vorzugsweise enthält R⁷ Struktureinheiten der Formel (3) 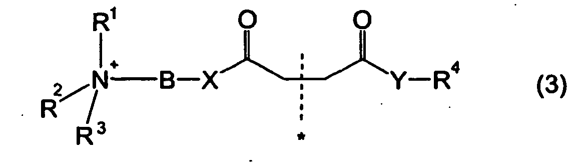 worin R¹, R², R³, R⁴, B, X und Y die bereits oben angegebenen Bedeutungen mit den jeweils oben für R¹, R², R³, R⁴, B, X und Y angegebenen Vorzugsbereichen haben. Die unterbrochene Linie bedeutet, dass die Struktureinheiten gemäß Formel (3) sowohl in 2- als auch in 3-Position der Dicarbonyl-Gruppe über eine Valenz eines Alkyl- bzw. Alkenylrests an beliebiger Stelle von R⁷ gebunden sein können.

M steht für ein ein- oder mehrwertiges Kation, vorzugsweise Metallion, besonders bevorzugt Alkali- oder Erdalkalimetallionen.

In einer weiteren bevorzugten Ausführungsform steht M für ein Ammoniumion der Formel N⁺R⁸R⁹R¹⁰R¹¹, worin R⁸, R⁹, R¹⁰ und R¹¹ unabhängig voneinander Wasserstoff oder einen beliebigen organischen Rest, der 1 bis 100 C-Atome enthält, und der gegebenenfalls Heteroatome enthalten kann, bedeuten. Enthalten R⁸, R⁹, R¹⁰ und/oder R¹¹ Heteroatome, so handelt es sich vorzugsweise um Stickstoff- oder Sauerstoffatome oder beides, vorzugsweise um beides. Die Stickstoffatome können in quaternierter Form vorliegen.

Steht R³ für C₁- bis C₂₂-Alkyl, C₂- bis C₂₂-Alkenyl bzw. C₆- bis C₃₀₋Alkylaryl, so sind als Gegenionen für die Verbindungen gemäß Formel (1) bis (3) alle Anionen geeignet, die die Löslichkeit der Verbindungen der Formel (1) bis (3) in den organisch-wässrigen Mischphasen nicht beeinträchtigen. Solche Gegenionen sind beispielsweise Methylsulfationen (Methosulfat) oder Halogenidionen.

Steht R³ für Reste -CHR⁵-COO⁻ bzw. -O⁻, so sind die Verbindungen der Formel (1) bis (3) Betaine bzw. Aminoxide und besitzen als innere Salze (Ampholyte) ein intramolekulares Gegenion.

Die erfindungsgemäßen Verbindungen können alleine oder in Kombination mit anderen bekannten Korrosionsinhibitoren und/oder Gashydratinhibitoren eingesetzt werden. Im allgemeinen wird man soviel des erfindungsgemäßen Korrosionsinhibitors und/oder Gashydratinhibitors einsetzen, dass man unter den gegebenen Bedingungen einen ausreichenden Korrosionsschutz und Schutz vor Gashydratbildung erhält.

Bevorzugte Einsatzkonzentrationen der Korrosionsinhibitoren bezogen auf die reinen erfindungsgemäßen Verbindungen sind 5 bis 5000 ppm, bevorzugt 10 bis 1000, insbesondere 15 bis 150 ppm.

Die Gashydratinhibitoren werden im allgemeinen in Mengen zwischen 0,01 und 5 Gew.-% der reinen erfindungsgemäßen Verbindungen bezogen auf die wässrige Phase, vorzugsweise zwischen 0,05 und 2 Gew.-% verwendet.

Besonders geeignet als Korrosionsinhibitoren und/oder Gashydratinhibitoren sind auch Mischungen der erfindungsgemäßen Produkte mit anderen Korrosionsinhibitoren und/oder Gashydratinhibitoren des Standes der Technik.

Besonders geeignet als Korrosionsinhibitoren und somit eine bevorzugte Ausführungsform dieser Erfindung sind Mischungen der Verbindungen gemäß Formel (1) bis (3), wie mit Amidaminen und/oder Imidazolinen aus Fettsäuren und Polyaminen und deren Salzen, quartären Ammoniumsalzen, oxethylierten/oxpropylierten Aminen, Amphoglycinaten und -propionaten, Betainen oder Verbindungen beschrieben in DE-A-19 930 683.

Besonders geeignet als Gashydratinhibitoren und somit eine bevorzugte Ausführungsform dieser Erfindung sind Mischungen der Verbindungen gemäß Formel (1) bis (3) mit einem oder mehreren Polymeren mit einem durch Polymerisation erhaltenen Kohlenstoff-Backbone und Amidbindungen in den Seitenketten. Hierzu zählen besonders Homopolymere und/oder Copolymere des Vinylpyrrolidons, Vinylcaprolactams, iso-Propylacrylamids, Acryloylpyrrolidins, N-Methyl-N-Vinylacetamid sowie weiterer anionischer, kationischer und neutraler Comonomere mit vinylischer Doppelbindung.

Werden Mischungen verwendet, so betragen die-Konzentrationsverhältnisse zwischen den erfindungsgemäßen Gashydratinhibitoren und den zugemischten Komponenten von 90:10 bis 10:90 Gewichtsprozente, vorzugsweise werden Mischungen in den Verhältnissen 75:25 bis 25:75, und insbesondere von 60:40 bis 40:60 verwendet.

Die erfindungsgemäßen Verbindungen können dadurch hergestellt werden, dass man substituierte Aminoalkohole oder substituierte Alkylendiamine mit Alkenylbernsteinsäurederivaten zu den entsprechenden Alkenylbersteinsäure-monooder -diestern bzw. -mono- oder -diamiden, gegebenenfalls zu zyklischen Alkenylbernsteinsäureimiden, gemäß den Umsetzungsverhältnissen und Reaktionsbedingungen umsetzt. Anschließend wird mit geeigneten Alkylierungsmitteln quaterniert.

Basis der verwendeten Aminoalkohole sind Dialkylamine mit C₁- bis C₂₂-Alkylresten oder C₂- bis C₂₂-Alkenylresten, bevorzugt C₃- bis C₈-Dialkylamine, die nach Stand der Technik in die entsprechenden Dialkylamino-alkohole überführt werden können. Geeignete Dialkylamine sind beispielsweise Di-n-butylamin, Diisobutylamin, Dipentylamin, Dihexylamin, Dioctylamin, Dicyclopentylamin, Dicyclohexylamin, Diphenylamin, Dibenzylamin.

Basis der verwendeten Alkylendiamine sind im wesentlichen Dialkylamino-alkylenamine mit C₁- bis C₂₂-Alkylresten oder C₂- bis C₂₂-Alkenylresten, bevorzugt tertiäre C₁- bis C₈-Dialkylamino-alkylenamine. Besonders geeignet sind beispielsweise N,N-Dibutylaminopropylamin, N,N-Diethylaminopropylamin, N,N-Dimethylamino-propylamin, N,N-Dimethylaminobutylamin, N,N-Dimethylaminohexylamin, N,N-Dimethylaminodecylamin, N,N-Dibutylaminoethylamin und N,N-Dimethylamino-2-hydroxypropylamin.

Die Herstellung Dialkylamino-alkylenamine ist im Stand der Technik beschrieben.

Basis der verwendeten Alkenylbernsteinsäurederivate sind die freien Disäuren, die Diester und die Anhydride. Besonders geeignet sind Alkenylbersteinsäureanhydride.

Die Herstellung von Alkenylbernsteinsäureanhydriden durch thermische oder katalysierte "En"-Reaktion ist im Stand der Technik beschrieben. Dabei werden Olefine, bevorzugt Olefine mit terminaler Doppelbindung, mit Maleinsäureanhydrid unter erhöhten Temperaturen umgesetzt. In Abhängigkeit von der Reaktionsführung, von der Art des verwendeten Olefins und vom verwendeten Molverhältnis werden Mono- und/oder Bisaddukte, gegebenenfalls Polyaddukte erhalten.

Die Alkenylbernsteinsäurederivate werden im allgemeinen mit den Aminoalkoholen bzw. Alkylendiaminen bei 60 - 240°C, bevorzugt bei 120 - 200°C derart umgesetzt, dass gegebenenfalls in Abhängigkeit vom verwendeten Alkenylbernsteinsäurederivat unter Eliminierung von Reaktionswasser oder von Alkohol eine vollständige Kondensation zu entsprechenden Mono- oder Diestern bzw. Mono- oder Dicarbonsäureamiden, gegebenenfalls zu zyklischen Imiden, erfolgt. Der Umsetzungsgrad kann durch die Bestimmung der Säurezahl, Verseifungszahl und/oder durch die Bestimmung des Basen- und/oder Amidstickstoffs verfolgt werden.

Die Umsetzung erfolgt in Substanz, kann aber auch bevorzugt in Lösung durchgeführt werden. Insbesondere bei der Verwendung von Alkenylbernsteinsäuren ist die Verwendung von Lösemitteln erforderlich, wenn hohe Umsätze und niedrigere Säurezahlen von den resultierenden Umsetzungsprodukten angestrebt werden. Geeignete Lösemittel für die Herstellung sind organische Verbindungen, durch die das Reaktionswasser azeotrop entfernt wird. Insbesondere können aromatische Lösemittel oder Lösemittelgemische, oder Alkohole verwendet werden. Besonders bevorzugt ist 2-Ethylhexanol. Die Reaktionsführung erfolgt dann beim Siedepunkt des Azeotrops.

Bei der Herstellung von Alkenylbernsteinsäurediamiden werden bevorzugt Alkenylbernsteinsäure-diester und ein Überschuss des entsprechenden Alkylendiamins verwendet, der mit dem frei werdenden Alkohol oder im Anschluss an die Umsetzung destillativ entfernt werden kann. Bei Verwendung von Alkenylbernsteinsäureanhydriden wird bevorzugt iterativ mit einem geeigneten Alkohol vollständig verestert und dann amidiert. Geeignete Alkohole sind z.B. Ethanol, Propanol, iso-Propanol oder 2-Ethylhexanol. Besonders bevorzugt ist 2-Ethylhexanol.

Bei der Herstellung der Amide fallen zwangsweise die entsprechenden zyklischen Alkenylbernsteinsäureimide als Nebenprodukte an, die umfaßt sind.

Gemäß dem Stand der Technik können die Veresterungen bzw. Amidierungen durch Zugabe geeigneter saurer Katalysatoren mit einem pKₐ-Wert von kleiner gleich 5, wie z.B. Mineralsäuren oder Sulfonsäuren, beschleunigt werden. Besonders bevorzugt sind Alkylstannansäuren.

Für die Herstellung der erfindungsgemäßen Verbindungen werden in einem anschließenden Reaktionsschritt die Alkenylbersteinsäure-mono- und -diester bzw.- mono- oder -diamide, gegebenenfalls die Alkenylbernsteinsäureimide, entsprechend quaterniert. Die Quaternierung kann durch entsprechende Alkylierungsmittel bei 50 bis 150°C erfolgen. Geeignete Alkylierungsmittel stellen Alkylhalogenide und Alkylsulfate dar, bevorzugt Methylchlorid, Methyljodid, Butylbromid und Dimethylsulfat.

Für die Herstellung der erfindungsgemäßen Betaine wird mit einer Halogencarbonsäure und einer Base, bevorzugt Chloressigsäure und Natriumhydroxid umgesetzt. Dies kann geschehen, indem man die Alkylaminoamide und/oder Alkylaminoimide mit 50 bis 125 Mol-% Halogencarbonsäure bei 40°C vorlegt und bei 40 bis 100°C durch einmalige oder portionierte Zugabe der Base und der zu 125 Mol-% verbleibenden Restmenge Halogencarbonsäure umsetzt.

Als basische Verbindungen können Erdalkali-/Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid, insbesondere deren wässrigen Lösungen.

Die Herstellung der erfindungsgemäßen Aminoxide erfolgt nach bekannten Verfahren des Standes der Technik, vorzugsweise durch Oxidation der entsprechenden tertiären Amingruppe mit Peroxiden oder Persäuren, bevorzugt mit Wasserstoffperoxid.

Die Umsetzungen zu den quatären Alkenylbersteinsäure-mono- oder -diestern bzw.- mono- oder -diamiden, gegebenenfalls zu zyklischen Alkenylbernsteinsäureimiden, erfolgen in Substanz, können aber auch bevorzugt in Lösung durchgeführt werden. Geeignete Lösemittel für die Herstellung der Quats, Betaine und Aminoxide sind inerte Alkohole wie Isopropanol, 2-Ethylhexanol oder inerte Ether wie Tetrahydrofuran, Glyme, Diglyme und MPEGs.

In Abhängigkeit von den gegebenen Anforderungen kann das verwendete Lösemittel im erfindungsgemäßen Produkt verbleiben oder muss destillativ entfernt werden.

### Beispiele:

### a) Allgemeine Vorschrift für die Herstellung der Alkylamino-alkylester aus Alkenylbernsteinsäureanhydriden

In einer Rührapparatur mit Rückflusskühler wurden 0,3 mol des entsprechenden Alkenylbernsteinsäureanhydrids (gemäß Verseifungszahl) unter Stickstoffspülung vorgelegt und auf 60°C erwärmt. Dann wurden 0,3 mol des entsprechenden Aminoalkohols über 0,5 Stunde zugetropft, wobei sich die Reaktionsmischung auf ca. 70°C erwärmte. Die Reaktionsmischung wurde 5 h bei 60°C nachgerührt.

### Beispiel 1 (Tetrapropylenbernsteinsäure-N,N-dibutylamino-N-ethylester)

Aus 87,8 g Tetrapropylenbernsteinsäureanhydrid (VZ = 383,3 mg KOH/g) und 52,0 g Dibutylaminethanol (DBAE; OHZ = 323,8 mg KOH/g) wurden 139 g Tetrapropylenbernsteinsäure-N,N-dibutylamino-N-ethylester mit SZ = 133,4 mg KOH/g und bas.-N = 2,93 % erhalten.

### Beispiel 2 (Pentapropylenbernsteinsäure-N,N-dibutylamino-N-ethylester)

Aus 117,4 g Pentapropylenbernsteinsäureanhydrid (VZ = 286,7 mg KOH/g) und 52,0 g Dibutylaminethanol (DBAE; OHZ = 323,8 mg KOH/g) wurden 169 g Pentapropylenbernsteinsäure-N,N-dibutylamino-N-ethylester mit SZ = 118,7 mg KOH/g und bas.-N = 2,45 % erhalten.

### Beispiel 3 (Polylsobutenylbernsteinsäure-N,N-dibutylamino-N-ethylester)

Aus 130,5 g Poiyisobutenyibernsteinsäureanhydrid (auf Basis PIB 550; VZ = 257,9 mg KOH/g) und 52,0 g Dibutylaminethanol (DBAE; OHZ = 323,8 mg KOH/g) wurden 182 g Polyisobutenylbernsteinsäure-N,N-dibutylamino-N-ethylester mit SZ = 106,1 mg KOH/g und bas.-N = 2,27 % erhalten.

### b) Allgemeine Vorschrift für die Herstellung der Alkylamino-alkyl-dieester aus Dicarbonsäureanhydriden

In einer Rührapparatur mit Destillationsbrücke wurden 0,3 mol des entsprechenden Alkenylbernsteinsäureanhydrids (gemäß Verseifungszahl) unter Stickstoffspülung vorgelegt und auf 60°C erwärmt. Dann wurden 0,6 mol des entsprechenden Aminoalkohols über 0,5 Stunde zugetropft, wobei sich die Reaktionsmischung auf ca. 70°C erwärmte. Die Reaktionsmischung wurde dann 0,5 h bei 60°C nachgerührt, anschließend auf 180°C erwärmt und bei dieser Temperatur 5 h Reaktionswasser abdestilliert. Abschließend wurde 2 h bei 200°C und 200 mbar das Reaktionswasser entfernt, bis eine Säurezahl (SZ) kleiner 10 mg KOH/g erreicht wurde.

### Beispiel 4 (Tetrapropylenbernsteinsäure-bis[N,N-dibutylamino-N-ethylester])

Aus 87,8 g Tetrapropylenbernsteinsäureanhydrid (VZ = 383,3 mg KOH/g) und 104,0 g Dibutylaminethanol (DBAE; OHZ = 323,8 mg KOH/g) wurden 172 g Tetrapropylenbernsteinsäure-bis[N,N-dibutylamino-N-ethylester] mit SZ = 7,1 mg KOH/g, VZ = 142,4 mg KOH/g und bas.-N = 4,34 % erhalten.

### Beispiel 5 (Pentapropylenbernsteinsäure-bis[N,N-dibutylamino-N-ethylester])

Aus 117,4 g Pentapropylenbernsteinsäureanhydrid (VZ = 286,7 mg KOH/g) und 104,0 g Dibutylaminethanol (DBAE; OHZ = 323,8 mg KOH/g) wurden 205 g Pentapropylenbernsteinsäure-bis[N,N-dibutylamino-N-ethylester] mit SZ = 9,6 mg, VZ = 100,3 mg KOH/g und bas.-N = 4,62 % erhalten.

### Beispiel 6 (Polyisobutenylbernsteinsäure-bis[N,N-dibutylamino-N-ethylester])

Aus 130,5 g Polyisobutenylbernsteinsäureanhydrid (auf Basis PIB 550; VZ = 257,9 mg KOH/g) und 104,0 g Dibutylaminethanol (DBAE; OHZ = 323,8 mg KOH/g) wurden 200 g Polyisobutenylbernsteinsäure-bis[N,N-dibutylamino-N-ethylester] mit SZ = 6,6 mg KOH/g, VZ = 117,5 mg KOH/g und bas.-N = 4,40 % erhalten.

### c) Allgemeine Vorschrift für die Quaternierung mit Dimethylsulfat

In einer Rührapparatur wurden 0,2 mol (nach bas.-N) des entsprechenden Amins unter Stickstoffspülung vorgelegt und auf 60 °C erwärmt. Dazu wurden 0,19 mol Dimethylsulfat so zugetropft, dass die Reaktionstemperatur 80-90°C nicht übersteigt. Die Reaktionsmischung wurde anschließend 3 h bei 90°C nachgerührt. Nach dieser Vorschrift wurden die Verbindungen, beschrieben durch die Beispiele 1 bis 6 quaternisiert (Beispiele 7 bis 12, wie in Tabelle 1 und 2 aufgeführt).

Wirksamkeit der erfindungsgemäßen Verbindungen als Korrosionsinhibitoren

Die erfindungsgemäßen Verbindungen wurden als Korrosionsinhibitoren im Shell-Wheel-Test geprüft. Coupons aus C-Stahl (DIN 1.1203 mit 15 cm² Oberfläche) wurden in eine Salzwasser/Petroleum-Mischung (9:1,5 %ige NaCl- Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

In den folgenden Tabellen bezeichnet "Vergleich 1" ein handelsübliches Rückstandsamin - Quat auf Basis Dicocosalkyl-dimethylammoniumchlorid und "Vergleich 2" ein handelsübliches Sojafettsäureamidopropyl-N,N-dimethylammonium-carboxymethyl-betain beschrieben durch EP-B-0 446 616 (Korrosionsinhibitoren des Standes der Technik).

**Tabelle 1: (SHELL-Wheel-Test)**

| Beispiel | Korrosionsinhibitor | Ø Schutz % |
|---|---|---|
| Vergleich 1 | Standard-Quat | 36,0 |
| Vergleich 2 | Standard-Betain | 75,4 |
| 7 | Quat aus Beispiel 1 | 87,6 |
| 8 | Quat aus Beispiel 2 | 90,1 |
| 9 | Quat aus Beispiel 3 | 92,4 |
| 10 | Quat aus Beispiel 4 | 70,5 |
| 11 | Quat aus Beispiel 5 | 84,1 |
| 12 | Quat aus Beispiel 6 | 89,3 |

Die Produkte wurden außerdem im LPR-Test (Testbedingungen analog ASTM D 2776) geprüft.

**Tabelle 2: (LPR-Test)**

| Beispiel | Korrosionsinhibitor | Schutz nach [%] | | |
|---|---|---|---|---|
| | | 10 min | 30 min | 60 min |
| Vergleich 1 | Standard-Quat | 53,9 | 61,2 | 73,7 |
| Vergleich 2 | Standard-Betain | 45,9 | 59,2 | 64,3 |
| 7 | Quat aus Beispiel 1 | 58,8 | 74,5 | 81,0 |
| 8 | Quat aus Beispiel 2 | 50,3 | 72,1 | 84,8 |
| 9 | Quat aus Beispiel 3 | 52,1 | 73,6 | 85,0 |
| 10 | Quat aus Beispiel 4 | 84,1 | 89,7 | 91,7 |
| 11 | Quat aus Beispiel 5 | 86,5 | 90,7 | 91,6 |
| 12 | Quat aus Beispiel 6 | 86,3 | 91,0 | 91,7 |

Wie aus den obigen Testresultaten zu erkennen ist, weisen die erfindungsgemäßen Produkte sehr gute Korrosionsschutzeigenschaften bei niedriger Dosierung auf und übertreffen im wesentlichen die Wirksamkeit der Inhibitoren des Standes der Technik. Die Verbindungen besitzen aufgrund ihrer Ester- bzw. Amidstruktur eine bessere biologische Abbaubarkeit und können mit einer niedrigeren Dosierrate eingesetzt werden.

## Patentansprüche

1. Verwendung von Verbindungen der Formel (1) 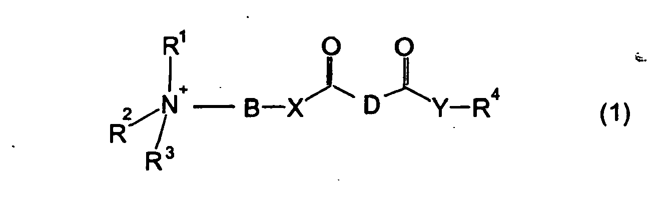 worin
R¹, R² unabhängig voneinander C₁- bis C₂₂-Alkyl, C₂- bis C₂₂-Alkenyl, C₆- bis C₃₀- Aryl- oder C₇- bis C₃₀-Alkylaryl, und,
R³ C₁- bis C₂₂-Alkyl, C₂- bis C₂₂-Alkenyl, C₆- bis C₃₀-Aryl- oder C₇- bis C₃₀- Alkylaryl,-CHR⁵-COO⁻ oder-O⁻,
R⁴ M, Wasserstoff oder einen gegebenenfalls Heteroatome enthaltenden organischen Rest mit 1 bis 100 Kohlenstoffatomen,
B eine gegebenenfalls substituierte C₁- bis C₁₀-Alkylengruppe,
D eine mit einem organischen Rest mit 1 bis 600 Kohlenstoffatomen substituierte Ethylengruppe,
X, Y unabhängig voneinander O oder NR⁶,
R⁵,R⁶ unabhängig voneinander Wasserstoff, C₁- bis C₂₂-Alkyl, C₂- bis C₂₂-Alkenyl, C₆- bis C₃₀-Aryl- oder C₇- bis C₃₀-Alkylaryl, und
M ein Kation
bedeuten, als Korrosions- und Gashydratinhibitoren.

2. Verwendung nach Anspruch 1, wobei B für eine C₂- bis C₄-Alkylengruppe steht.

3. Verwendung nach Anspruch 1 und/oder 2, wobei R¹ und R² unabhängig voneinander für eine Alkyl- oder Alkenylgruppe von 2 bis 14 Kohlenstoffatomen stehen.

4. Verwendung nach einem oder mehreren der Ansprüche 1 bis 3, wobei R³ für eine Alkyl- oder Alkenylgruppe mit 1 bis 12 Kohlenstoffatomen steht.

5. Verwendung nach einem oder mehreren der Ansprüche 1 bis 4, wobei R⁵ und R⁶ für Wasserstoff stehen.

6. Verwendung nach einem oder mehreren der Ansprüche 1 bis 5, wobei R⁴ für einen Rest der Formel (2) steht 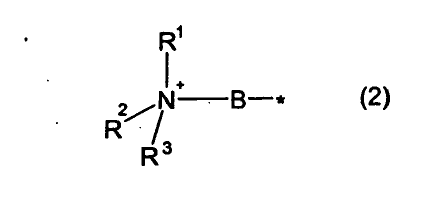 worin R¹, R², R³ und B die in Anspruch 1 angegebenen Bedeutungen haben.

7. Verwendung nach einem oder mehreren der Ansprüche 1 bis 6, wobei D für eine Struktureinheit der Formel 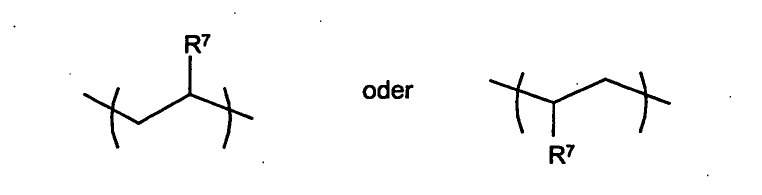 steht, in der R⁷ für C₂- bis C₁₀₀-Alkyl- oder Alkenylreste steht.

8. Verwendung nach einem oder mehreren der Ansprüche 1 bis 7, wobei R⁷ für Struktureinheiten der Formel (3) steht 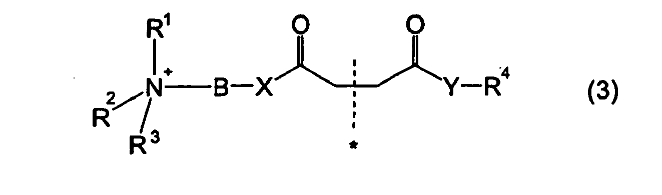 worin R¹, R², R³, R⁴, B, X und Y die in Anspruch 1 angegebenen Bedeutungen haben.

9. Verbindungen der Formel (1) 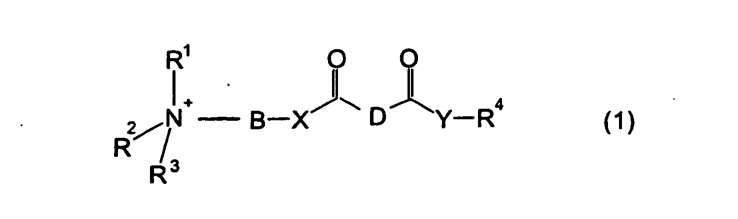 worin
R¹, R² unabhängig voneinander C₁- bis C₂₂-Alkyl, C₂- bis C₂₂-Alkenyl, C₆- bis C₃₀- Aryl- oder C₇- bis C₃₀-Alkylaryl, und,
R³ C₁- bis C₂₂-Alkyl, C₂- bis C₂₂-Alkenyl, C₆- bis C₃₀-Aryl- oder C₇- bis C₃₀- Alkylaryl,-CHR⁵-COO⁻ oder-O⁻,
R⁴ M, Wasserstoff oder einen gegebenenfalls Heteroatome enthaltenden organischen Rest mit 1 bis 100 Kohlenstoffatomen,
B eine gegebenenfalls substituierte C₁- bis C₁₀-Alkylengruppe,
D eine mit einem organischen Rest mit 1 bis 600 Kohlenstoffatomen substituierte Ethylengruppe,
X, Y unabhängig voneinander O oder NR⁶,
R⁵, R⁶ unabhängig voneinander Wasserstoff, C₁- bis C₂₂-Alkyl, C₂- bis C₂₂-Alkenyl, C₆- bis C₃₀-Aryl- oder C₇- bis C₃₀-Alkylaryl, und
M ein Kation
bedeuten.

## Claims

1. The use of compounds of the formula (1) 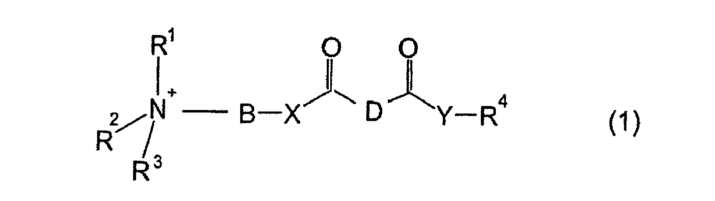 where
R¹, R² are each independently C₁- to C₂₂-alkyl, C₂- to C₂₂-alkenyl, C₆- to C₃₀-aryl or C₇- to C₃₀- alkylaryl,
R³ is C₁- to C₂₂-alkyl, C₂- to C₂₂-alkenyl, C₆- to C₃₀- aryl or C₇- to C₃₀-alkylaryl, -CHR⁵-COO⁻ or -O⁻,
R⁴ is M, hydrogen or an organic radical which optionally contains heteroatoms and has from 1 to 100 carbon atoms,
B is an optionally substituted C₁- to C₁₀-alkylene group,
D is an ethylene group substituted by an organic radical having from 1 to 600 carbon atoms,
X, Y are each independently O or NR⁶,
R⁵, R⁶ are each independently hydrogen, C₁- to C₂₂- alkyl, C₂- to C₂₂-alkenyl, C₆- to C₃₀-aryl or C₇- to C₃₀-alkylaryl, and
M is a cation
as corrosion and gas hydrate inhibitors.

2. The use as claimed in claim 1, wherein B is a C₂- to C₄-alkylene group.

3. The use as claimed in claim 1 and/or 2, wherein R¹ and R² are each independently an alkyl or alkenyl group of from 2 to 14 carbon atoms.

4. The use as claimed in one or more of claims 1 to 3, wherein R³ is an alkyl or alkenyl group having from 1 to 12 carbon atoms.

5. The use as claimed in one or more of claims 1 to 4, wherein R⁵ and R⁶ are hydrogen.

6. The use as claimed in one or more of claims 1 to 5, wherein R⁴ is a radical of the formula (2) 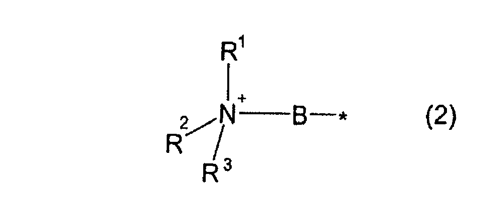 where R¹, R², R³ and B are each as defined in claim 1.

7. The use as claimed in one or more of claims 1 to 6, wherein D is a structural unit of the formula 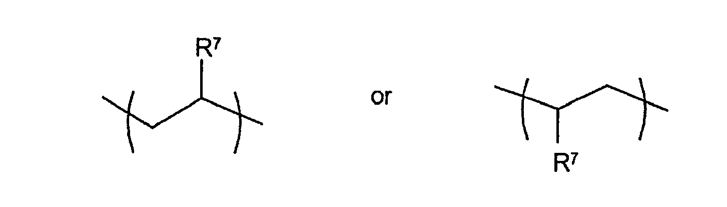 in which R⁷ is C₂ - to C₁₀₀-alkyl or alkenyl radicals.

8. The use as claimed in one or more of claims 1 to 7, wherein R⁷ is structural units of the formula (3) 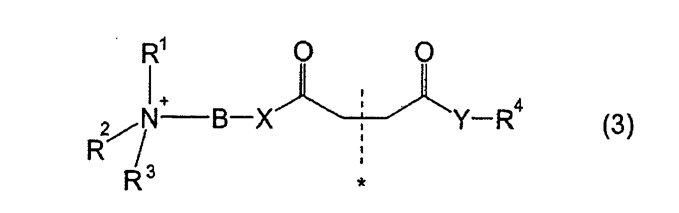 where R¹, R², R³, R⁴, B, X and Y are each as defined in claim 1.

9. A compound of the formula (1) 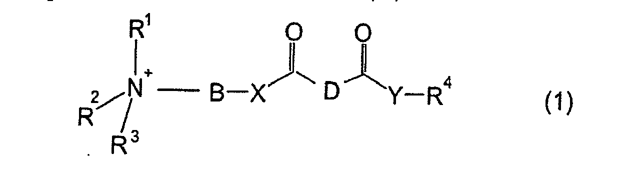 where
R¹, R² are each independently C₁- to C₂₂-alkyl, C₂- to C₂₂-alkenyl, C₆- to C₃₀-aryl or C₇- to C₃₀- alkylaryl,
R³ is C₁- to C₂₂-alkyl, C₂- to C₂₂-alkenyl, C₆- to C₃₀- aryl or C₇- to C₃₀-alkylaryl, -CHR⁵-COO⁻ or -O⁻,
R⁴ is M, hydrogen or an organic radical which optionally contains heteroatoms and has from 1 to 100 carbon atoms,
B is an optionally substituted C₁- to C₁₀-alkylene group,
D is an ethylene group substituted by an organic radical having from 1 to 600 carbon atoms,
X, Y are each independently O or NR⁶,
R⁵, R⁶ are each independently hydrogen, C₁- to C₂₂- alkyl, C₂- to C₂₂-alkenyl, C₆- to C₃₀-aryl or C₇- to C₃₀-alkylaryl, and
M is a cation.

## Revendications

1. Utilisation de composés de formule (1) 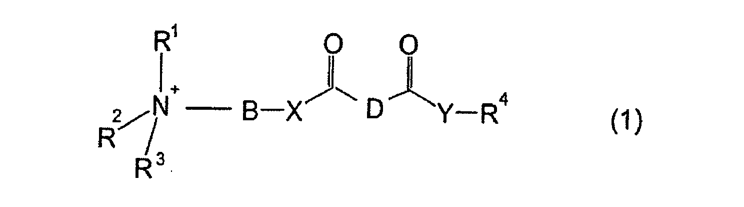 dans laquelle
R¹, R² représentent, indépendamment l'un de l'autre, un groupe alkyle en C₁-C₂₂, alcényle en C₂-C₂₂, aryle en C₆-C₃₀ ou alkylaryle en C₇-C₃₀, et
R³ représente un groupe alkyle en C₁-C₂₂, alcényle en C₂-C₂₂, aryle en C₆-C₃₀ ou alkylaryle en C₇-C₃₀, -CHR⁵-COO⁻ ou -O⁻,
R⁴ représente M, un atome d'hydrogène ou un radical organique ayant de 1 à 100 atomes de carbone, contenant éventuellement des hétéroatomes,
B représente un groupe alkylène en C₁-C₁₀ éventuellement substitué,
D représente un groupe éthylène substitué par un radical organique ayant de 1 à 600 atomes de carbone,
X, Y représentent, indépendamment l'un de l'autre, O ou NR⁶,
R⁵, R⁶, représentent, indépendamment l'un de l'autre, un atome d'hydrogène, un groupe alkyle en C₁-C₂₂, alcényle en C₂-C₂₂, aryle en C₆-C₃₀ ou alkylaryle en C₇-C₃₀, et
M représente un cation,
en tant qu'agents anticorrosion et inhibiteurs de la formation d'hydrates de gaz.

2. Utilisation selon la revendication 1, dans laquelle B représente un groupe alkylène en C₂-C₄,

3. Utilisation selon la revendication 1 et/ou la revendication 2, dans laquelle R¹ et R² représentent, indépendamment l'un de l'autre, un groupe alkyle ou alcényle ayant de 2 à 14 atomes de carbone.

4. Utilisation selon une ou plusieurs des revendications 1 à 3, dans laquelle R³ représente un groupe alkyle ou alcényle ayant de 1 à 12 atomes de carbone.

5. Utilisation selon une ou plusieurs des revendications 1 à 4, dans laquelle R⁵ et R⁶ représentent un atome d'hydrogène.

6. Utilisation selon une ou plusieurs des revendications 1 à 5, dans laquelle R⁴ représente un radical de formule (2) 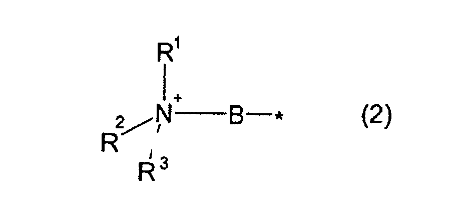 dans laquelle R¹, R², R³ et B ont les significations indiquées dans la revendication 1.

7. Utilisation selon une ou plusieurs des revendications 1 à 6, dans laquelle D représente une unité structurale de formule 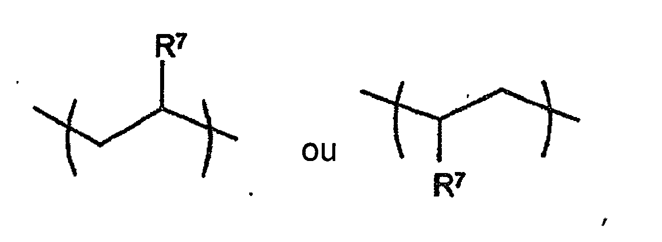 formules dans lesquelles R⁷ représente des radicaux alkyle ou alcényle en C₂-C₁₀₀.

8. Utilisation selon une ou plusieurs des revendications 1 à 7, dans laquelle R ⁷ représente des unités structurales de formule (3) 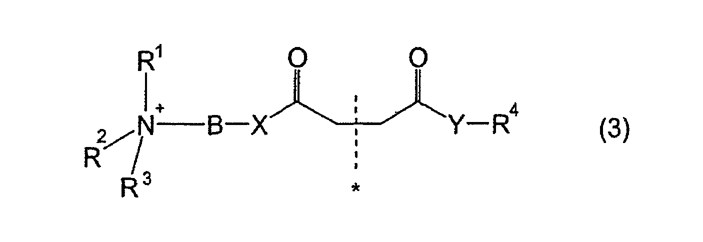 dans laquelle R¹, R², R³, R⁴, B, X et Y ont les significations indiquées dans la revendication 1.

9. Composés de formule (1) 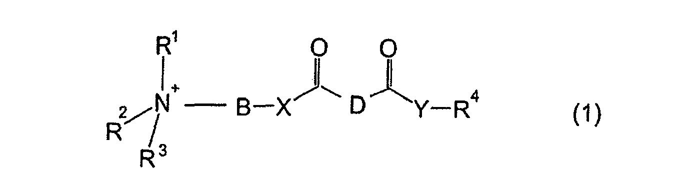 dans laquelle
R¹, R² représentent, indépendamment l'un de l'autre, un groupe alkyle en C₁-C₂₂, alcényle en C₂-C₂₂, aryle en C₆-C₃₀ ou alkylaryle en C₇-C₃₀, et
R³ représente un groupe alkyle en C₁-C₂₂, alcényle en C₂-C₂₂, aryle en C₆-C₃₀ ou alkylaryle en C₇-C₃₀, -CHR⁵-COO⁻ ou -O⁻,
R⁴ représente M, un atome d'hydrogène ou un radical organique ayant de 1 à 100 atomes de carbone, contenant éventuellement des hétéroatomes,
B représente un groupe alkylène en C₁-C₁₀ éventuellement substitué,
D représente un groupe éthylène substitué par un radical organique ayant de 1 à 600 atomes de carbone,
X, Y représentent, indépendamment l'un de l'autre, O ou NR⁶,
R⁵, R⁶ représentent, indépendamment l'un de l'autre, un atome d'hydrogène, un groupe alkyle en C₁-C₂₂, alcényle en C₂-C₂₂, aryle en C₆-C₃₀ ou alkylaryle en C₇-C₃₀, et
M représente un cation.
